# FASCICULE DE BREVET EUROPEEN

(11) **EP 1 302 460 B1**
(45) Date de publication et mention de la délivrance du brevet: **02.08.2006**
(21) Numéro de dépôt: 03000188.7
(22) Date de dépôt: 26.08.1998
(51) Int. Cl.: C07D 209/42, C07D 209/60, A61K 31/40, A61P 35/00

(54) **Composés indole-carboxyliques et leur utilisation comme composés pharmaceutiques**
Indolecarbonsäurederivate und ihre Verwendung als Arzneimittel
Indolecarboxylic compounds and their use as pharmaceutical compounds

(30) Priorité: 05.09.1997 FR 9711076
(43) Date de publication de la demande: 16.04.2003
(62) Demande divisionnaire de: 98942786.9
(73) Titulaire: L'ORÉAL, 75008 Paris (FR)
(72) Inventeur: Bernard, Bruno, 92200 Neuilly Sur Seine (FR); Gerst, Catherine, 92600 Asnieres/Seine (FR); Galey, Jean-Baptiste, 93600 Aulnay-Sous-Bois (FR); Dalko, Maria, 91190 Gif/Yvette (FR); Pichaud, Patrick, 91190 Gif Sur Yvette (FR)
(74) Mandataire: Allab, Myriam

(56) Documents cités:
- WO-A-94/22821
- HOLT, DENNIS A. ET AL: "Benzophenone- and Indolecarboxylic Acids: Potent Type-2 Specific Inhibitors of Human Steroid 5.alpha.-Reductase" J. MED. CHEM. (1995), 38(1), 13-15, 1995, XP002068506

## Description

La présente invention concerne l'utilisation d'une quantité efficace d'au moins un composé de la famille des indole-carboxyliques pour traiter les désordres liés à une suractivité de la 5α-réductase et plus particulièrement les désordres androgéno-dépendants.

Les androgènes sont des hormones définies comme appartenant à la famille des stéroïdes possédant une structure spécifique.

Les androgènes agissent à de nombreux sites du corps humains et interviennent par là même dans un grand nombre de désordres parmi lesquels on peut citer les carcinomes prostatiques, l'hyperplasie bénigne de la prostate, l'acné, l'hirsutisme, la séborrhée, l'alopécie androgénique, les chéloïdes et les adhésions, le syndrome polycyclique ovarien, le syndrome prémenstruel, le cancer du poumon chez l'homme, la puberté précoce, la maladie de Fox et Fordyce.

Les androgènes sont des substances lipidiques qui traversent facilement les membranes cellulaires. Le mécanisme d'action des androgènes se fait par l'interaction avec un récepteur qui leur est spécifique : le récepteur aux androgènes.

La testostérone apparaît comme l'androgène majeur. Les voies métaboliques des androgènes, de la testostérone en particulier, sont aujourd'hui bien connues.
Une des voies métaboliques de la testostérone est sa conversion par la 5α-réductase en dihydrotestostérone (DHT). La testostérone et la DHT se lient au récepteur aux androgènes, mais la DHT présente une affinité beaucoup plus élevée pour ce récepteur que la testostérone. De plus la liaison DHT/récepteur est beaucoup plus stable que la liaison testostérone/récepteur.

Deux isoformes de la 5α-réductase ont été à ce jour isolées et clonées. La 5α-réductase de type 1 est majoritairement exprimée dans la peau et les différents compartiments du follicule pileux particulièrement dans les kératinocytes de l'épiderme et/ou du follicule, dans les cellules de la papille dermique, la gaine externe du follicule pileux, la glande sébacée et dans les glandes sudoripares.
La 5α-réductase de type 2 est plutôt exprimée dans l'épididyme, les vésicules séminales, la prostate, la peau génitale foetale ou encore la gaine interne du follicule pileux ou dans les fibroblastes de la peau génitale adulte.

La 5α-réductase de type 1 étant majoritairement exprimée dans la peau et les différents compartiments du follicule pileux, le développement d'inhibiteurs de la 5α-réductase de type 1 représente une approche de choix pour traiter les désordres androgéno-dépendants, qu'ils se manifestent au niveau de la peau ou du follicule pileux.

Depuis longtemps on a cherché à développer des inhibiteurs de la 5α-réductase comme traitement des désordres liés aux androgènes. Dans cet ordre d'idée de nombreux composés ont été proposés. A ce jour, deux classes d'inhibiteurs ont été synthétisées : les inhibiteurs stéroïdiens et les inhibiteurs non-stéroïdiens (voir à cet égard l'article de Chen et col. "The 5α-reductase system and its inhibitors" (Dermatology 1996 ; 193 : 177-184)).
Parmi les inhibiteurs stéroïdiens on peut citer les dérivés 6-azasteroides et surtout les dérivés 4-azasteroides, dont le [17β-(N-tert-butylcarbamoyl)-4-aza-5α-an-drostan-1-en-3-one] (finastéride), un inhibiteur spécifique de l'isotype 2 et le 7β-méthyl-4-aza-cholestan-3-one (MK-386) un inhibiteur spécifique de l'isotype 1. L'inconvénient des inhibiteurs stéroïdiens est qu'ils présentent des effets secondaires non-négligeables qui rendent leur utilisation délicate.

Parmi les inhibiteurs non-stéroïdiens on peut citer certains dérivés de l'acide benzoylaminophénoxybutanoique, des benzoquinolines tels que letrans-8-chloro-4-methyl-1,2,3,4,4a,5,6,10b-octahydrobenzo[*f*]quinolin-3-one (LY191704), le 4-[3-(-[bis(4-isobutylphenyl)methylamino]-benzoyl)-1H-indol-1-yl]butyric acid (FK143), des acides gras polyinsaturés, des cations tels que le cuivre et le zinc, et des dérivés de l'épicatéchine. De même il a été décrit des dérivés d'indole comme par exemple dans les demandes EP-458207, EP-511477, EP-600084, EP-628040, WO-9113060, WO-9302050, WO-9302051, WO-9305019, WO-9316996, WO-9427990, WO-9505375, WO-9523143, JP-07304736, JP-07188164.

La classe des dérivés indole-carboxyliques a été peu décrite. Holt et collaborateurs (J. Med. Chem., 1995, 38: 13-15) ont décrit des molécules qui présentent une forte activité inhibitrice vis-à-vis de la 5α-réductase de type 2.

Ainsi, un premier objet de l'invention concerne l'utilisation dans une composition, d'une quantité efficace d'au moins un composé répondant à la formule générale (I) 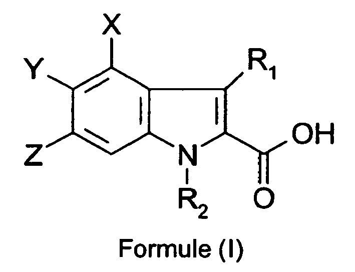 dans laquelle :
- X représente un atome d'hydrogène ;
- Y représente un radical -O-CHR₃R₄,
   dans lequel R₃ est un atome d'hydrogène ou un radical phényle éventuellement substitué ou encore un hétérocycle ayant 5 ou 6 chaînons et R₄ est un radical phényle éventuellement substitué ou encore un hétérocycle ayant 5 ou 6 chaînons, ou R₃ et R₄ pris ensemble forment avec l'atome de carbone un cycle ou un hétérocycle ayant 5 ou 6 chaînons ;
- Z représente un radical -O-R₅ dans lequel R₅ est un radical alkyle en C₁-C₄ choisi parmi les radicaux méthyle, éthyle, propyle, isopropyle, butyle, isobutyle ou tertiobutyle ;
R₁ représente un atome d'hydrogène;
R₂ représente un atome d'hydrogène;
étant entendu que lorsque R₂ est un atome d'hydrogène et X est un atome d'hydrogène alors Y doit être différent du radical - O-CH₂Phényl et Z différent d'un atome d'hydrogène, et que lorsque R₂ est un atome d'hydrogène et X est un atome d'hydrogène alors Y doit être différent d'un atome d'hydrogène et Z différent du radical - O-CH₂Phényl et que R, X, Y et Z ne peuvent être simultanément un atome d'hydrogène,
le composé ou la composition étant destinés à traiter les désordres liés aux androgènes et/ou à une suractivité de la 5α-réductase de type 2.

Les composés de l'invention répondant à la formule (I) peuvent être par exemple utilisés pour traiter les carcinomes prostatiques, l'hyperplasie bénigne de la prostate, l'acné, l'hirsutisme, la séborrhée, et l'alopécie androgénique.
Préférentiellement, les composés répondant à la formule (I) selon l'invention peuvent être utilisés pour induire et/ou stimuler la croissance des cheveux et/ou freiner leur chute et/ou dans le traitement de l'hyperséborrhée et/ou de l'acné.

Particulièrement, l'invention concerne l'utilisation dans une composition, d'une quantité efficace d'au moins un composé répondant à la formule générale (I) tel que défini ci-dessus, ce composé ou la composition étant destinés à traiter les désordres liés à une suractivité de la 5α-réductase de type 2.

Ainsi, l'invention à pour objet l'utilisation dans une composition, d'une quantité efficace d'au moins un composé répondant à la formule générale (I) tel que défini ci-dessus, ce composé ou la composition étant destinés à traiter raciné, l'hirsutisme, la séborrhée, l'alopécie androgénique, les carcinomes prostatiques, l'hyperplasie bénigne de la prostate.
Préférentiellement, l'invention a pour objet l'utilisation dans une composition, d'une quantité efficace d'au moins un composé répondant à la formule générale (I) tel que défini ci-dessus, ce composé la composition étant destinés à induire et/ou stimuler la croissance des cheveux et/ou freiner leur chute et/ou à traiter l'hyperséborrhée et/ou l'acné.

Bien entendu, les composés répondant à la formule (I) selon l'invention peuvent être utilisés seuls ou en mélange.

Parmi les composés utilisés préférentiellement selon l'invention on peut citer
l'acide 5 benzyloxy 6 méthoxy 1 H indole 2 carboxylique,
l'acide 5-(3,5-bis-trifluorométhyl-benzyloxy)-6-méthoxy-1 H-indole-2-carboxylique,
l'acide 5-(3,4,5-triméthoxy-benzyloxy)-6-méthoxy -1 H-indole-2-carboxylique,
l'acide 5,6 bis benzyloxy 3 méthyl 1H indole 2 carboxylique,
l'acide 5,6 bis benzyloxy 1H indole 2 carboxylique,
l'acide 5-(4-méthoxy-benzyloxy)-6-méthoxy-1 H-indole-2-carboxylique,
l'acide 5-(4-cyano-benzyloxy)-6-méthoxy-1 H-indole-2-carboxylique,
l'acide 1-(3,5-bis-trifluorométhyl-benzyl)-5-(3,5-trifluorométhyl-benzyloxy)-6-méthoxy-1 H-indole-2-carboxylique,
un dérivé selon la formule ii) pour lequel Y représente un radical -OCH₂Ph, Z représente un radical -OMe, R₁ et R₂ représentent un atome d'hydrogène;
un dérivé pour lequel Y et Z représentent un radical -OCH₂Ph, R₁ et R₂ représente un hydrogène.

Parmi ces composés, on préfère tout particulièrement :
l'acide 5 benzyloxy 6 méthoxy 1 H indole 2 carboxylique,
l'acide 5-(3,5-bis-trifluorométhyl-benzyloxy)-6-méthoxy-1 H-indole-2-carboxylique,
l'acide 5,6 bis benzyloxy 1 H indole 2 carboxylique,

La quantité de composés de formule (I) utilisable selon l'invention est bien entendu fonction de l'effet recherché et peut donc varier dans une large mesure.

Pour donner un ordre de grandeur, le composé de formule (I) peut être utilisé selon l'invention en une quantité représentant de 0,001 % à 10% du poids total de la composition et préférentiellement en une quantité représentant de 0,01% à 5% du poids total de la composition.

Selon l'invention, les composés de formule (I) peuvent être utilisés dans des compositions à usage cosmétique ou pharmaceutique. Préférentiellement selon l'invention, les composés de formule (I) sont utilisés dans des compositions à usage cosmétique

Dans le traitement de la chute des cheveux, comme dans celui de la séborrhée ou de l'acné, la composition cosmétique selon l'invention est à appliquer sur les zones à traiter et est éventuellement laissée en contact plusieurs heures et est éventuellement à rincer. On peut, par exemple, appliquer la composition contenant une quantité efficace d'au moins un composé tel que défini précédemment, le soir, garder celle-ci au contact toute la nuit et éventuellement effectuer un rinçage le matin. Ces applications peuvent être renouvelées quotidiennement pendant un ou plusieurs mois suivant les individus.

Ainsi, la présente invention a pour deuxième objet un procédé de traitement cosmétique des cheveux et/ou du cuir chevelu et/ou la peau, caractérisé par le fait qu'il consiste à appliquer sur les cheveux et/ou le cuir chevelu et/ou la peau, une composition cosmétique comprenant une quantité efficace d'au moins un composé répondant à la formule (I), à laisser celle-ci au contact des cheveux et/ou du cuir chevelu et/ou de la peau, et éventuellement à rincer.

Le procédé de traitement présente les caractéristiques d'un procédé cosmétique dans la mesure où il permet d'améliorer l'esthétique des cheveux et/ou de la peau en leur donnant une plus grande vigueur et un aspect amélioré.

Ainsi, les indole-carboxyliques présentent des activités remarquables qui justifient leur utilisation pour traiter les désordres liés aux androgènes et/ou à une suractivité de la 5α-réductase.

A cet égard, après de long travaux la demanderesse a découvert de nouveaux dérivés indole-carboxyliques qui sont des inhibiteurs spécifiques de ia 5α-réductase de type 1 ou de type 2, ou qui peuvent être considérés comme des inhibiteurs mixtes c'est à dire présentant une activité inhibitrice marquée sur les deux formes.

Ainsi, l'invention a pour troisième objet un dérivé d'indole-carboxylique répondant à la formule générale (I') : 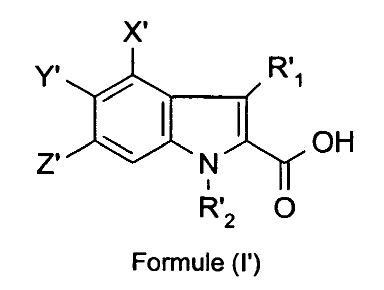 dans laquelle :
- X' représente un atome d'hydrogène ;
- Y' représente un un radical -O-CHR'₃R'₄,dans lequel R'₃ est un atome d'hydrogène ou un radical phényle éventuellement substitué ou encore un hétérocycle ayant 5 ou 6 chaînons et R'₄ est un radical phényle éventuellement substitué ou encore un hétérocycle ayant 5 ou 6 chaînons, ou R'₃ et R'₄ pris ensemble forment avec l'atome de carbone un cycle ou un hétérocycle ayant 5
ou 6 chaînons ;

Z' représente un radical -O-R'₅ dans lequel R'₅ est un radical alkyle en C₁-C₄ choisi parmi les radicaux méthyle, éthyle, propyle, isopropyle, butyle, isobutyle ou tertiobutyle ;
R'₁ représente un atome d'hydrogène;
R'₂ représente un atome d'hydrogène;
ou ses esters ou ses isomères optiques, seuls ou en mélange en toutes proportions, ses formes acylées ou encore ses sels pharmaceutiquement acceptables.
étant entendu que Y' ne peut être un radical - OCHR'₃R'₄ dans lequel CHR'₃R'₄ représente un radical aralkyle, alors Z' ne peut être un radical -OR'₅.

Par hétérocycle, on entend de préférence selon l'invention un cycle incluant éventuellement un ou plusieurs atomes d'azote et/ou d'oxygène et particulièrement la pyridine, l'imidazole, le tétrahydrofuranne ou le furanne. Un hétérocycle particulièrement préféré selon l'invention est la pyridine.

Par radical alkyle en C₁ - C₆, on entend de préférence selon l'invention les radicaux alkyles linéaires ou ramifiés, saturés ou insaturés, ayant de 1 à 6 atomes de carbone.
Préférentiellement selon l'invention, le radical alkyle est en C₁-C₄ et est choisi parmi les radicaux méthyle, éthyle, propyle, isopropyle, butyle, isobutyle ou tertiobutyle et plus particulièrement les radicaux méthyle ou éthyle.

Par radical aralkyle en C₆ - C₁₂, on entend de préférence selon l'invention les radicaux alkyles-aryles ayant de 6 à 12 atomes de carbone, définition dans laquelle le terme aryle s'entend comme un cycle aromatique ayant 5 ou 6 chaînons ou un hétérocycle aromatique ayant 5 ou 6 chaînons. Préférentiellement selon l'invention, le radical aralkyle est en C₇-C₁₀. Un radical aralkyle particulièrement préféré selon l'invention est le radical benzyle.

Par un radical phényle substitué, on entend de préférence selon l'invention le radical phényle substitué par un groupement cyano (-CN), un groupement trifluorométhyle (-CF₃), un radical méthoxy (-O-CH₃) ou un atome d'halogène. L'atome d'halogène peut être choisi parmi le chlore, le brome, le fluor, l'iode. Un radical phényle substitué particulièrement préféré selon l'invention est le radical phényle substitué par un groupement trifluorométhyle (-CF₃).

De préférence lorsque R₃ ou R₄ indépendamment l'un de l'autre sont un radical phényle substitué, celui-ci est substitué par un groupement cyano (-CN), un groupement trifluorométhyle (-CF₃), un radical méthoxy (-O-CH₃) ou un atome d'halogène.Lorsque R'₃ est un radical phényle substitué, R'₃ est préférentiellement un radical di-3,5-(trifluorométhyl)phényle.

Lorsque R'₃ est un hétérocycle, R'₃ est préférentiellement une pyridine.

Lorsque R'₄ est un radical phényle substitué, R'₄ est préférentiellement un radical di-3,5-(trifluorométhyl)phényle.

Lorsque R'₄ est un hétérocycle, R'₄ est préférentiellement une pyridine.

Selon une forme de réalisation particulière de l'invention, R'₅ est préférentiellement un radical méthyle.

Selon une autre forme de réalisation de invention, R'₅ est un radical éthyle.

On peut citer comme composés de formule (I') :
l'acide 5-(3,5-bis-trifluorométhyl-benzyloxy)-6-méthoxy-1H-indole-2-carboxylique,
l'acide 5-(3,4,5-triméthoxy-benzyloxy)-6-méthoxy -1 H-indole-2-carboxylique,
l'acide 5-(4-méthoxy-benzyloxy)-6-méthoxy-1 H-indole-2-carboxylique,
l'acide 5-(4-cyano-benzyloxy)-6-méthoxy-1 H-indole-2-carboxylique,
l'acide 1-(3,5-bis-trifluorométhyl-benzyl)-5-(3,5-trifluorométhyl-benzyloxy)-6-méthoxy-1 H-indole-2-carboxylique,
un dérivé pour lequel Y' représente un radical -OCH₂Ph, Z' représente un radical -OMe, R₁ et R₂ représentent un atome d'hydrogène;
un dérivé pour lequel Y' et Z' représentent un radical -OCH₂Ph, R₁ et R₂ représente un hydrogène.

Parmi ces composés, on préfère tout particulièrement :
l'acide 5-(3,5-bis-trifluorométhyl-benzyloxy)-6-méthoxy-1 H-indole-2-carboxylique,

Un quatrième objet de l'invention concerne des compositions cosmétiques ou pharmaceutiques, particulièrement dermatologiques, qui comprennent au moins un des composés répondant à la formule (I') définis ci-dessus.

Bien entendu les compositions selon l'invention peuvent comprendre les composés de formule (I') seuls ou en mélanges en toutes proportions.

La quantité de composés de formule (I') contenue dans les compositions de l'invention est bien entendu fonction de l'effet recherché et peut donc varier dans une large mesure.

Pour donner un ordre de grandeur, la composition de l'invention peut contenir au moins un composé de formule (I') en une quantité représentant de 0,001 % à 10% du poids total de la composition et préférentiellement en une quantité représentant de 0,01% à 5% du poids total de la composition.

La composition de l'invention peut être une composition à usage cosmétique ou pharmaceutique. Préférentiellement selon l'invention, La composition de l'invention peut être une composition à usage cosmétique;

La composition peut être ingérée, injectée ou appliquée sur la peau (sur toute zone cutanée du corps), les cheveux, les ongles ou les muqueuses (buccale, jugale, gingivale, génitale, conjonctive). Selon le mode d'administration, la composition selon l'invention peut se présenter sous toutes les formes galéniques normalement utilisées.

Pour une application topique sur la peau, la composition peut avoir la forme notamment de solution aqueuse ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol comprenant également un agent propulseur sous pression.
La composition selon l'invention peut aussi être une composition pour soins capillaires, et notamment un shampooing, une lotion de mise en plis, une lotion traitante, une crème ou un gel coiffant, une composition de teintures (notamment teintures d'oxydation) éventuellement sous forme de shampooings colorants, des lotions restructurantes pour les cheveux, une composition de permanente (notamment une composition pour le premier temps d'une permanente), une lotion ou un gel antichute, un shampooing antiparasitaire, etc.

Pour l'injection, la composition peut se présenter sous forme de lotion aqueuse, huileuse ou sous forme de sérum. Pour les yeux, elle peut se présenter sous forme de gouttes et pour l'ingestion, eiie peut se présenter sous forme de capsules, de granulés de sirops ou de comprimés.

Les quantités des différents constituants des compositions selon l'invention sont celles classiquement utilisées dans les domaines considérés.

Les compositions selon l'invention peuvent également consister en des préparations solides constituant des savons ou des pains de nettoyage.

Les compositions peuvent aussi être conditionnées sous forme d'une composition pour aérosol comprenant également un agent propulseur sous pression.

Lorsque la composition est une émulsion, la proportion de la phase grasse peut aller de 5% à 80% en poids, et de préférence de 5% à 50% en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3% à 30% en poids, et de préférence de 0,5 à 20% en poids par rapport au poids total de la composition. L'émulsion peut, en outre, contenir des vésicules lipidiques.

Lorsque la composition est une solution ou un gel huileux, la phase grasse peut représenter plus de 90% du poids total de la composition.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01% à 10% du poids total de la composition. Ces adjuvants, selon leur nature, peuvent être introduits dans la phase grasse, dans la phase aqueuse et/ou dans les sphérules lipidiques.
Comme huiles ou cires utilisables dans l'invention, on peut citer les huiles minérales (huile de vaseline), les huiles végétales (fraction liquide du beurre de karité, huile de tournesol), les huiles animales (perhydrosquaiène), ies huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).
Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Selon l'invention la composition peut associer au moins composé de formule (I) à d'autres agents actifs. Parmi ces agents actifs, on peut citer à titre d'exemple :
- les agents améliorant l'activité sur la repousse et/ou sur le freinage de la chute des cheveux, et ayant déjà été décrits pour cette activité comme par exemple les esters d'acide nicotinique, dont notamment le nicotinate de tocophérol, le nicotinate de benzyle et les nicotinates d'alkyles en C₁-C₆ comme les nicotinates de méthyle ou d'hexyle, les dérivés de pyrimidine, comme le 2,4-diamino 6-piperidinopyrimidine 3-oxyde ou "Minoxidil" décrit dans les brevets US 4 139 619 et US 4 596 812, les agents favorisant la repousse des cheveux comme ceux décrits par la demanderesse dans la demande de brevet européen publiée sous le numéro 0648488 ;
- les agents diminuant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens comme par exemple l'ibuprofène et ses sels, le diclofénac et ses sels, l'acide acétylsalicylique, l'acétaminophène ou l'acide glycyrrhizique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides α- et β-hydroxycarboxyliques ou β-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'α-tocophérol ou ses esters, les superoxyde dismutases, certains chélatants de métaux ou l'acide ascorbique et ses esters ;
- les anti-séborrhéiques tels que la progestérone ;
- les antipelliculaires comme l'octopirox ou la pyrithione de zinc ;
- les antiacnéiques comme l'acide rétinoïque ou le peroxyde de benzoyle ;
- les extraits d'origine végétale ou bactérienne.

A la liste ci-dessus, d'autres composés peuvent également être rajoutés, à savoir par exemple le Diazoxyde, la Spiroxazone, des phospholipides comme la lécithine, les acides linoléique et linolénique, l'acide salicylique et ses dérivés décrits dans le brevet français FR 2 581 542, comme les dérivés de l'acide salicylique porteurs d'un groupement alcanoyle ayant de 2 à 12 atomes de carbone en position 5 du cycle benzénique, des acides hydroxycarboxyliques ou cétocarboxyliques et leurs esters, des lactones et leurs sels correspondants, l'anthraline, des caroténoides, les acides eicosatétraénoïque et eicosatriénoïque ou leurs esters et amides, la vitamine D et ses dérivés, des extraits d'origine végétale ou bactérienne.

Ainsi, selon un mode particulier, la composition selon l'invention comprend également au moins un agent choisi parmi les agents antibactériens, les antiparasitaires, les antifongiques, les antiviraux, les anti-inflammatoires, les antiprurigineux, les anesthésiques, les kératolytiques, les anti-radicaux libres, les anti-séborrhéiques, les antipelliculaires, les antiacnéiques et/ou les agents diminuant la différenciation et/ou la prolifération et/ou la pigmentation cutanée, les extraits d'origine végétale ou bactérienne.

On peut également envisager que la composition comprenant au moins un composé tel que défini précédemment soit sous forme Liposomé, telle que notamment décrite dans la demande de brevet WO 94/22468 déposée le 13 octobre 1994 par la société Anti Cancer Inc. Ainsi, le composé encapsulé dans les liposomes peut être délivré sélectivement au niveau du follicule pileux.

Lorsque la composition selon l'invention est une composition pharmaceutique, elle peut être administrée par voie parentérale, entérale ou encore par voie topique. De préférence, la composition pharmaceutique est administrée par voie topique.

Un cinquième objet de l'invention concerne l'utilisation à titre de médicament des composés de formule générale (1').

Les composés de l'invention peuvent être synthétisés par des procédés tout à fait classiques généralement utilisés en synthèse organique.
Des exemples détaillés de ces synthèses sont donnés par ailleurs dans les exemples.
Mais à titre d'exemple et de manière très générale, pour obtenir un composé de l'invention substitué en position 4,6, on fait dans une première étape réagir du 2-hydroxy-4-alkoxy benzaldéhyde de formule 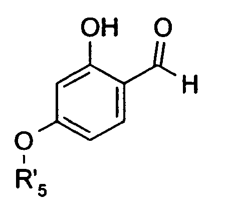 avec un bromure de formule

Br-R

dans laquelle R représente un atome d'hydrogène ou un radical O-CHR'₃R'₄ tel que défini précédemment, et R'₅ est tel que défini précédemment en présence de tetrahydrofurane (THF) anhydre, d'hydrure de sodium à 60% dans l'huile et d'iodure de tétrabutylammonium.
Après 6 heures d'incubation à température ambiante, le composé obtenu est purifié par chromatographie sur gel de silice.
Par température ambiante on entend un température comprise entre 18°C et 35°C préférentiellement, comprise entre 20°C et 30°C.

Dans une deuxième étape le composé obtenu à la première étape est mis à réagir en présence de méthylate de sodium et d'azido acétate d'éthyle en présence de méthanol pendant 20 heures à température ambiante.
Le composé ainsi obtenu est purifié par chromatographie sur gel de silice ou par recristallisation.

Dans une troisième étape le composé obtenu à la seconde étape est mis à réagir en présence de toluène, à reflux pendant une heure et demi. Le composé ainsi obtenu est purifié par précipitation.

Dans une quatrième étape, le composé obtenu à la troisième étape est mis à réagir en présence de méthanol, d'acétone et de soude pendant 4 heures. Le composé ainsi obtenu est purifié par précipitation.

De même à titre d'exemple et de manière très générale pour obtenir un composé de l'invention substitué en position 5,6, on réalise dans une première étape la nitration du 3-aralcoxy-4-alcoxybenzadehyde de formule 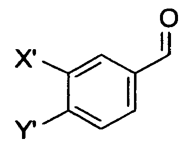 dans laquelle X' et Y' sont définis comme précédemment, par l'acide nitrique à température ambiante, pendant un temps compris entre 2h et 12h.
Le composé obtenu est isolé par filtration.

Dans une deuxième étape le composé obtenu à la 1^{ère} étape est mis à réagir en présence de carbonate de calcium (K₂CO₃) avec du phosphonate de triéthyle dans un mélange d'éthanol et diméthoxyéthane à 80°C pendant un temps compris entre 10 à 20 heures. Le composé obtenu est purifié par chromatographie sur colonne de silice.

Dans une troisième étape le composé obtenu à la 2^{iéme} étape est mis à réagir avec du triéthyle phosphate au reflux pendant un temps compris entre 2 et 6 heures. Le composé obtenu est purifié par chromatographie sur colonne de silice.

Dans une quatrième étape le composé obtenu à la 3^{ième} étape est saponifié par la soude dans un mélange acétone / méthanol pendant un temps compris entre 1 à 6 heures à une température comprise entre 20°C et 40°C. Le composé obtenu est purifié par cristallisation.

Pour obtenir un composé pour lequel X' et Y' forment un cycle ou un hétérocycle on fait dans une première étape réagir un aldéhyde aromatique bicyclique en présence de méthylate de sodium, d'azido acétate d'éthyle, et de méthanol.

Après plusieurs heures d'incubation à température ambiante, le composé obtenu est purifié par chromatographie sur colonne de silice.

Dans une deuxième étape le composé obtenu à la première étape est mis à réagir en présence de toluène, à reflux pendant plusieurs heures. Le composé ainsi obtenu est purifié par recristallisation.

Dans une troisième étape le composé obtenu à la seconde étape est mis à réagir en présence de méthanol, d'acétone et de soude pendant 4 heures.
Le composé ainsi obtenu est purifié par recristallisation.

Pour la préparation des composés dans lesquels R'₂ n'est pas un atome d'hydrogène, le composé obtenu à la seconde étape est mis en présence d'un halogénure d'alkyle pendant 30 minutes à température ambiante. Le composé alors obtenu est ensuite mis à réagir en présence d'acétone et de soude pendant 1 heure à température ambiante. Le composé ainsi obtenu est purifié par recristallisation.

Les indole-carboxyliques présentent des activités inhibitrices de la 5α-réductase qui ont pu être mises en évidence grâce à un test de criblage basé sur l'expression in vitro des isoformes 1 ou 2 de la 5α-réductase.
Les détails de ce tests et les résultats obtenus sont présentés dans les exemples.

On va maintenant donner à titre d'illustration des exemples qui ne sauraient limiter en aucune façon la portée de l'invention.

Les composés des exemples 1, 2, 7 et 8 ne font pas partie de l'invention.

### Exemple n°1 : synthèse de l'acide 4-benzyloxy-6-méthoxy-1 H-indole-2-carboxylique

### a) Synthèse du 2-benzyloxy-4-méthoxy-benzaldéhyde de formule :

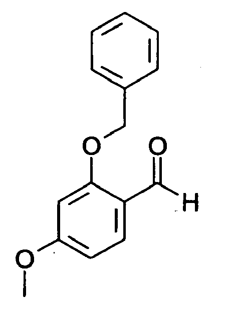

Dans un réacteur, on solubilise 1 g de 2-hydroxy-4-méthoxy benzaldéhyde dans 15 ml de tétrahydrofuranne (THF) anhydre. On refroidit à 4°C. On ajoute 1,3 équivalents d'hydrure de sodium (NaH) à 60% dans l'huile. On laisse réagir 30 minutes pour former l'alcoolate. On ajoute ensuite 1,3 équivalents de bromure de benzyle, et 90 mg d'iodure de tétrabutylammonium. On laisser réagir pendant 6 heures à la température ambiante.
Après 6h de réaction, on ajoute 50 ml d'une solution saturée en bicarbonate de sodium (NaHCO₃). On fait deux lavages par 50 ml d'éther isopropylique. On sèche les phases organiques sur sulfate de sodium et évaporation sous vide et on purifie le composé obtenu sur gel de silice en utilisant le dichlorométhane comme éluant. La masse récupérée est de 1,1 g ce qui correspond à un rendement de 69%.

### b) Synthèse de l'ester méthylique de l'acide 2-azido-3-(2-benzyloxy-4-méthoxy-phényl)-acrylique de formule :

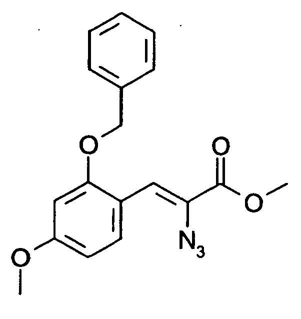

Dans un tricol parfaitement sec, on introduit 2 équivalents de méthylate de sodium en poudre dans 10 ml de méthanol, le tout sous argon. On verse ensuite 1,1 g de 2-benzyloxy-4-méthoxy benzaldéhyde préalablement solubilisés dans 2 ml de méthanol.
On refroidit à -10°C. On solubilise 5 équivalents d'azido acétate d'éthyle dans 5 ml de méthanol, et on verse lentement sur la solution de 2-benzyloxy-4-méthoxy benzaldéhyde. On laisse réagir 20 heures après retour à la température ambiante. On dilue le milieu par 100 ml de dichlorométhane, et on fait deux lavages par 50 ml d'eau. On sèche les phases organiques sur sulfate de sodium et évaporation sous vide et on purifie le composé obtenu sur gel de silice en utilisant l'heptane comme éluant (gradient d'acétate d'éthyle (AcOEt) jusqu'à 10%).
La masse récupérée est de 0,25 g, ce qui correspond à un rendement de 15%.

### c) Synthèse de l'ester méthylique de l'acide 4-benzyloxy-6-méthoxy-1 H-indole-2-carboxylique de formule :

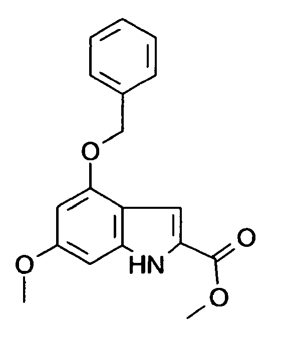

On solubilise 0,25 g de l'ester méthylique de l'acide 2-azido-3-(2-benzyloxy-4-méthoxy-phényl)-acrylique dans 10 ml de toluène. On porte au reflux. Après 1 h30, on concentre le milieu à sec sous vide et on lave le solide obtenu par 20 ml d'heptane. On obtient un solide. La masse récupérée est de 0,11 g, ce qui correspond à un rendement de 50%.

### d) Synthèse de l'acide 4-benzyloxy-6-méthoxy-1 H-indole-2-carboxylique de formule:

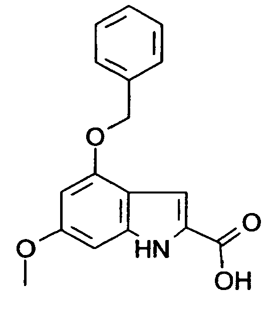

On solubilise 0,11 g de l'ester méthylique de l'acide 4-benzyloxy-6-méthoxy-1 H-indole-2-carboxylique dans 3 ml d'acétone et 2 ml de méthanol. On ajoute ensuite 6 ml de soude 1 N. On laisse réagir 4 heures à température ambiante. On concentre le milieu au rotavapor, et on dilue par 20 ml d'eau. On acidifie à pH = 2 par de l'acide chlorhydrique concentré. On obtient un solide marron clair. La masse récupérée est de 700 mg, ce qui correspond à un rendement de 67%.

Les analyses en RMN ¹H (200MHz ; CDCl₃) δ ppm ont donné les résultats suivants :
3.66 (3H ; s), 5.0 (2H ; s), 6.1 (1H ; s), 6.27 (1H ; s), 7.25 (6H ; m), 8.66 (1H ; s) La structure du composé obtenu est conforme à la structure attendue.

### Exemple n°2 : synthèse de l'acide 4-(3,5-bis-trifluorométhyl-benzyloxy)-6-méthoxy-1 H-indole-2-carboxylique :

### a) Synthèse du 2-(3,5-bis-trifluorométhyl-benzyloxy)-4-méthoxy-benzaldéhyde de formule :

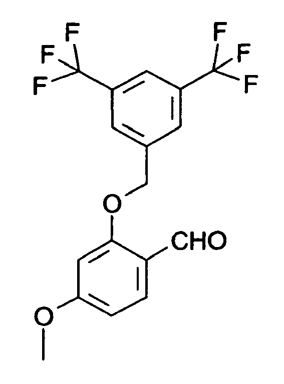

Dans un réacteur, on pèse 1,1 équivalents d'hydrure de sodium (NaH) en dispersion dans l'huile. On ajoute 20 ml de diméthylformamide (DMF) anhydre. On ajoute ensuite 0,95 équivalent de 2-hydroxy-4-méthoxy benzaldéhyde pour former l'alcoolate. On laisser 30 minutes à température ambiante.
On ajoute 4,2 g de bromure de (3,5-bis-trifluorométhyle) de benzyle. On laisse réagir 1h30 à température ambiante. On verse 25 ml d'une solution saturée bicarbonate de sodium (NaHCO₃) et on fait deux extractions par 20 ml de dichlorométhane. On sèche les phases organiques sur sulfate de sodium et on les concentre sous vide afin d'obtenir une huile légèrement rosée. On reprend cette dernière dans 15 ml d'heptane, puis dans 20 ml d'éther isopropylique. On obtient 3,6 g de solide blanc cassé, ce qui correspond à un rendement de 74%.

### b) Synthèse de l'ester méthylique de l'acide 2-azido-3-[2-(3,5-bis-trifluorométhyl-benzyloxy)-méthoxy-phényl]-acrylique de formule :

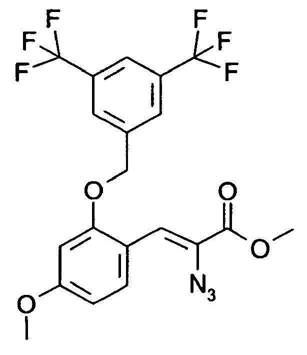

Dans un réacteur parfaitement séché à l'étuve, on pèse 1 g de méthylate de sodium en poudre. On ajoute 10 ml de méthanol, puis 3,5 g de 2-(3,5-bis-trifluorométhyl-benzyloxy)-4-méthoxy-benzaldéhyde préalablement solubilisés dans 5 ml de méthanol. On refroidit à 0°C.
On solubilise 4 équivalents d'azido acétate d'éthyle dans 5 ml de méthanol, et on verse ensuite sur le milieu précédent. On laisser réagir au retour à la température ambiante pendant 30 heures.
On dilue le milieu par 20 ml de dichlorométhane, et on fait deux lavages par 50 ml d'eau. On sèche la phase organique sur sulfate de sodium. On concentre à sec sous vide et on purifie l'huile obtenue sur colonne de silice en utilisant le dichlorométhane comme éluant et en faisant un gradient de méthanol (jusqu'à 2%). La masse récupérée est de 700 mg, ce qui correspond à un rendement de 12%.

### c) Synthèse de l'ester méthylique de l'acide 4-(3,5-bis-trifluorométhyl-benzyloxy)-6-méthoxy-1 H-indole-2-carboxylique de formule :

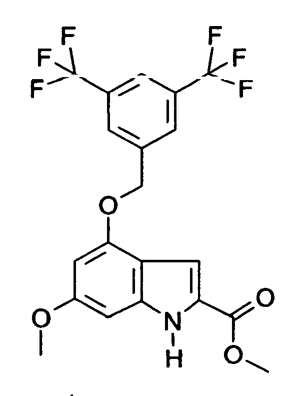

On solubilise 700 mg de l'ester méthylique de l'acide 2-azido-3-[2-(3,5-bis-trifluorométhyl-benzyloxy)-4-méthoxy-phényl]-acrylique dans 25 ml de toluène. On porte à reflux pendant 3 heures. On concentre le milieu à sec sous vide. On obtient un solide jaune clair. On reprend ce dernier dans 10 ml d'éther isopropylique. La masse récupérée est de 460 mg, ce qui correspond à un rendement de 83%.

### d) Synthèse de l'acide 4-(3,5-bis-trifluorométhyl-benzyloxy)-6-méthoxy-1 H-indole-2-carboxylique de formule :

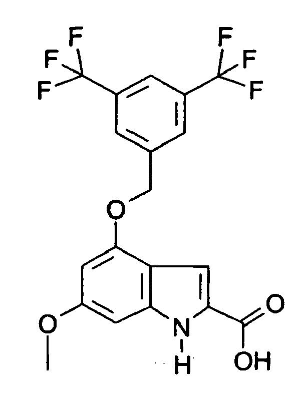

On solubilise 450 mg de l'ester méthylique de l'acide 4-(3,5-bis-trifluorométhyl-benzyloxy)-6-méthoxy-1H-indole-2-carboxylique dans 15 ml d'acétone, puis on ajoute 15 ml de soude 1N. On laisse agiter 4 heures à la température ambiante. On évapore l'acétone sous vide. On dilue le milieu par 10 ml d'eau, et on neutralise à pH = 7 par de l'acide chlorhydrique concentré. On obtient 300 mg de solide blanc ce qui correspond à un rendement de 69%.

Les analyses en RMN ¹H (200MHz ; DMSO) δ ppm ont donné les résultats suivants :
3.75 (3H ; s), 5.42 (2H ; s), 6.28 (1H ; s), 6.51 (1H ; s), 6.95 (1H ; s), 8.1 (1H ; s), 8.2(2H;s),11.5(1H;s)
La structure du composé obtenu est conforme à la structure attendue.

### Exemple n°3 : synthèse de l'acide 5-(3,5-bis-trifluorométhyl-benzyloxy)-6-méthoxy-1 H-indole-2-carboxylique :

### a) Synthèse de l'ester éthylique de l'acide 5-hydroxy-6-méthoxy-1 H-indole-2-carboxylique de formule :

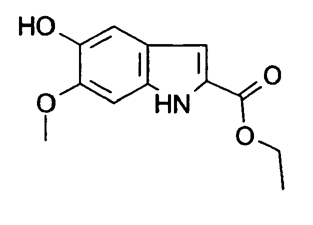

On solubilise 4,8 g de l'ester éthylique de l'acide 5-benzyloxy-6-méthoxy-1 H-indole-2-carboxylique, dans 150 ml d'acétate d'éthyle (AcOEt) et 30 ml d'éthanol. On ajoute 4,8 g de Palladium activé (10%) sur charbon et on effectue la débenzylation sous 5 bars d'hydrogène pendant 2h30. On filtre le catalyseur sur célite et on concentre à sec le milieu sous vide. On obtient un solide légèrement gris clair.
La masse récupérée est de 2,55 g, ce qui correspond à un rendement de 72%.

### b) Synthèse de l'ester éthylique de l'acide 5-(3,5-bis-trifluorométhyl-benzyloxy)-6-méthoxy-1 H-indole-2-carboxylique

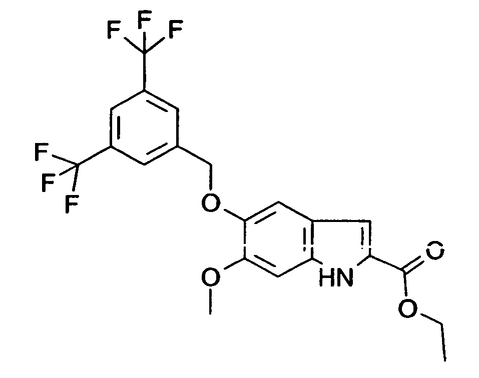

Dans un réacteur préalablement séché à l'étuve, on introduit 1,1 équivalents d'hydrure de sodium (NaH) sous argon, et 5 ml de diméthylformamide (DMF) anhydre. On ajoute ensuite 0,3 g de l'ester éthylique de l'acide 5-hydroxy-6-méthoxy-1H-indole-2-carboxylique à +3°C, et on laisse réagir 30' à cette température, afin de former l'alcoolate.
On ajoute ensuite 1,05 équivalents de bromure de 3,5-bis-trifluorométhyle benzyle, toujours à +3°C. On laisse réagir 1h30 à cette température. On ajoute 25 ml d'une solution saturée en bicarbonate de sodium (NaHCO₃) sous agitation pendant 1 heure. On lave deux fois par 50 ml de dichlorométhane. Les phases organiques sont séchées sur sulfate de sodium et concentrées à sec sous vide. Le résidu obtenu est purifié sur colonne de silice en utilisant le dichlorométhane comme solvant.
La masse récupérée est de 220 mg, ce qui correspond à un rendement de 37%.

### c) Synthèse de l'acide 5-(3,5-bis-trifluorométhyl-benzyloxy)-6-méthoxy-1 H-indole-2-carboxylique de formule :

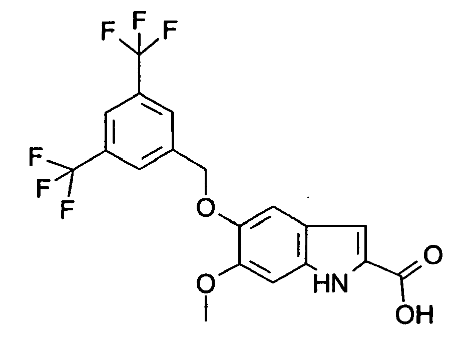

On solubilise 220 mg de l'ester éthylique de l'acide 5-(3,5-bis-trifluorométhyl-benzyloxy)-6-méthoxy-1H-indole-2-carboxylique dans 10 ml d'acétone et 5 ml de méthanol. On ajoute 15 ml de soude 1N, et on laisse réagir 3 heures à 30°C. On concentre les solvants sous vide, puis on dilue par 5 ml d'eau. On acidifie à pH = 2 par de l'acide chlorhydrique concentré. On obtient un précipité que l'on lave par de l'eau. On reprend le solide encore humide dans 50 ml de pentane à reflux pendant 1 heure. On filtre sous vide et on obtient 70 mg de solide pour un rendement de 34%.

Les analyses en RMN ¹H (200MHz ; DMSO) δ ppm ont donné les résultats suivants :
3.81 (3H ; s), 5.27 (3H ; s), 5.27 (2H ; s), 6.91 (1H ; s), 6.95 (1H ; s), 7.24 (1H ; s), 8.08 (1H ; s), 8.17 (2H ; s), 11.53 (1H ; s), 12.65 (1H ; s)
La structure du composé obtenu est conforme à la structure attendue.

| Analyse Elémentaire : | | | | |
|---|---|---|---|---|
| | C en % | H en % | N en % | F en % |
| théorique | 52,67 | 3,02 | 3,23 | 26,31 |
| trouvée | 52,09 | 3,11 | 3,11 | 26,44 |

### Exemple n°4 : synthèse de l'acide 5-(3,4,5-triméthoxy-benzyloxy)-6-méthoxy-1 H-indole-2-carboxylique :

### a) Synthèse de l'ester éthylique de l'acide 5-(3,4,5-triméthoxy-benzyloxy)-6-méthoxy-1 H-indole-2-carboxylique de formule :

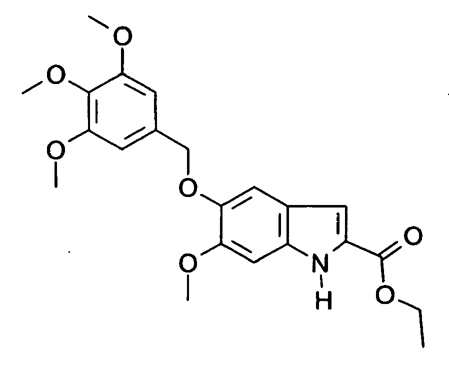

Dans un réacteur préalablement séché à l'étuve, on introduit 1,1 équivalents d'hydrure de sodium (NaH) sous argon et 5 ml de diméthylformamide (DMF) anhydre. On ajoute ensuite 0,3 g de l'ester éthylique de l'acide 5-hydroxy-6-méthoxy-1 H-indole-2-carboxylique à +3°C, et on laisse réagir 30 minutes à cette température afin de former l'alcoolate.
On ajoute ensuite 1,05 équivalents de chlorure de 3,4,5-triméthoxybenzyle, toujours à +3°C. On laisse réagir 1h30 à cette température. On ajoute 10% en mole de iodure de tétrabutylammonium et on laisse réagir 2 heures supplémentaires. On ajoute alors 25 ml d'une solution saturée en bicarbonate de sodium (NaHCO3) sous agitation pendant 1 heure. On lave deux fois par 50 ml de dichlorométhane. Les phases organiques sont séchées sur sulfate de sodium et concentrées à sec sous vide. Le résidu obtenu est purifié sur colonne de silice en utilisant le dichlorométhane comme solvant.
La masse récupérée est de 200 mg, ce qui correspond à un rendement de 38%.

### b) Synthèse de l'acide 5-(3,4,5-triméthoxy-benzyloxy)-6-méthoxy-1H-indole-2-carboxylique de formule :

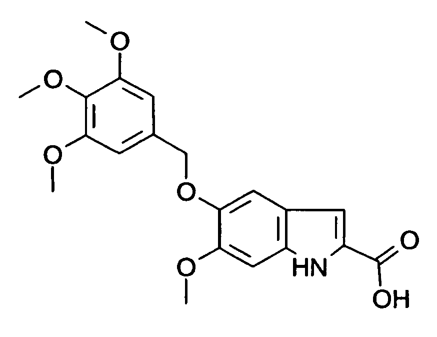

On solubilise 180 mg de l'ester méthylique de l'acide 5-(3,4,5-triméthoxy-benzyloxy)-6-méthoxy-1H-indole-2-carboxylique dans 15 ml d'acétone et 3 ml de méthanol. On ajoute 15 ml de soude 1N, et on laisse réagir pendant 2 heures à 30°C. On évapore les solvants sous vide et on acidifie la phase aqueuse résiduelle à pH = 2, par de l'acide chlorhydrique concentré. On obtient un solide que l'on purifie par reprise dans l'acétate d'éthyle à température ambiante.
La masse récupérée est de 35 mg, ce qui correspond à un rendement de 21%.

Les analyses en RMN ¹H (200MHz ; CDCl₃) δ ppm ont donné les résultats suivants :
3.6 (3H ; s), 3.85 (9H ; s), 4.95 (2H ; s), 6.7 (1H ; s), 6.8 (2H ; s), 6.9 (1H ; s), 7.5 (1H ; s), 11.05 (1H ; se)
La structure du composé obtenu est conforme à la structure attendue.

### Exemple n°5 : synthèse de l'acide 5-(4-méthoxy-benzyloxyF6-méthoxy-1H-indole -2- carboxylique :

### a) Synthèse de l'ester éthylique de l'acide 5-(4-méthoxy-benzyloxy)-6-méthoxy-1 H-indole-2-carboxylique de formule :

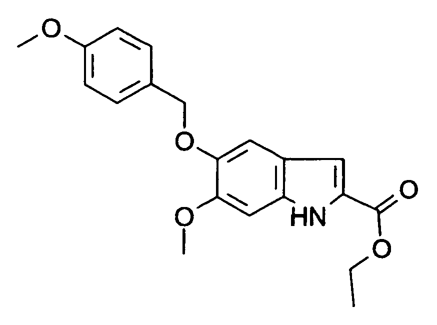

Dans un réacteur préalablement séché à l'étuve, on introduit 1,1 équivalents d'hydrure de sodium (NaH) sous argon et 5 ml de diméthylformamide (DMF) anhydre. On ajoute ensuite 0,4 g de l'ester éthylique de l'acide 5-hydroxy-6-méthoxy-1H-indole-2-carboxylique à 0°C, et on laisse réagir 30 minutes à cette température, afin de former l'alcoolate. On ajoute ensuite 1,1 équivalents de chlorure de 4-méthoxybenzyle et 10% en mole d'iodure de tétrabutylammonium. On laisse réagir jusqu'au retour à la température ambiante. La réaction est complète après 24 heures. On verse ensuite 50 ml d'une solution saturée en bicarbonate de sodium (NaHCO₃) et on extrait par deux fois 50 ml de dichlorométhane. On sèche la phase organique sur sulfate de sodium, on la concentre à sec sous vide, et on purifie le résidu obtenu sur colonne de silice en utilisant le dichlorométhane comme éluant. La masse récupérée est de 200 mg, ce qui correspond à un rendement de 32%.

### b) Synthèse de l'acide 5-(4-méthoxy-benzyloxy)-6-méthoxy-1 H-indole-2-carboxylique de formule :

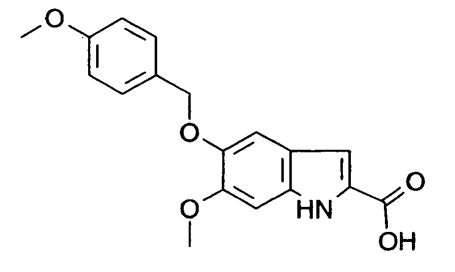

Dans un réacteur, on solubilise 0,2 g d'ester éthylique de l'acide 6-méthoxy-5-(4-méthoxy-benzyloxy)-1 H-indole-2-carboxylique dans 15 ml d'acétone. On ajoute 15 ml de soude 1N, et on laisse réagir à la température ambiante pendant 6 heures. On évapore le solvant sous vide puis on neutralise la phase aqueuse résiduelle par addition d'acide chlorhydrique concentré jusqu'à obtention d'un pH de 6. On filtre le solide obtenu et on reprend ce dernier dans l'éther isopropylique. On filtre et on sèche le solide au dessiccateur sous vide. La masse récupérée est de 0,05 g, ce qui correspond à un rendement de 30%.

Les analyses en RMN ¹H (200MHz ; DMSO) δ ppm ont donné les résultats suivants :
3.8 (6H ; d), 5.1 (2H ; s), 6.9 (4H ; m), 7.2 (1H ; s), 7.4 (2H ; d), 11.6 (1H ; s), 12.7 (1H; s).
La structure du composé obtenu est conforme à la structure attendue.

### Exemple n°6 : synthèse de l'acide 5-(4-cyano-benzyloxy)-6-méthoxy-1 H-indole-2-carboxylique :

### a) Synthèse de l'ester éthylique de l'acide 5-(4-cyano-benzyloxy)-6-méthoxy-1 H-indole-2-carboxylique de formule:

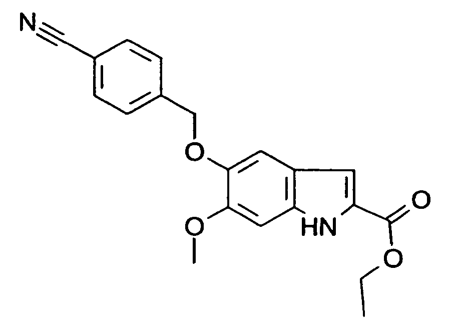

Dans un réacteur préalablement séché à l'étuve, on introduit 1,6 équivalents d'hydrure de sodium (NaH) sous argon et 5 ml de diméthylformamide (DMF) anhydre. On ajoute ensuite 0,3 g de l'ester éthylique de l'acide 5-hydroxy-6-méthoxy-1 H-indole-2-carboxylique à 0°C, et on laisse réagir 30 minutes à cette température afin de former l'alcoolate. On ajoute ensuite 1,3 équivalents de bromure de 4-cyano benzyle et on laisse réagir jusqu'au retour à la température ambiante, avec agitation pendant 1 h30 . On verse ensuite 50 ml d'une solution saturée en bicarbonate de sodium (NaHCO₃) et on extrait par deux fois 50 ml de dichlorométhane. On sèche la phase organique sur sulfate de sodium, on la concentre à sec sous vide et on purifie le résidu obtenu sur colonne de silice en utilisant le dichlorométhane comme éluant. La masse récupérée est de 230 mg, ce qui correspond à un rendement de 50%.

### b) Synthèse de l'acide 5-(4-cyano-benzyloxy)-6-méthoxy-1 H-indole-2-carboxylique de formule:

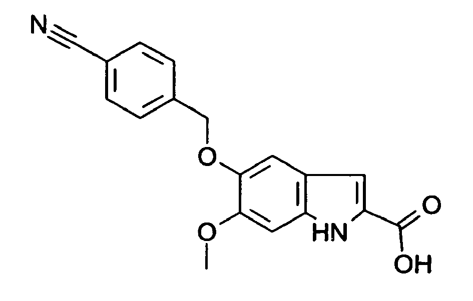

Dans un réacteur, on solubilise 0,230 g d'ester éthylique de l'acide 5-(4-cyano-benzyloxy)-6-méthoxy-1H-indole-2-carboxylique dans 10 ml d'acétone et 5 ml de méthanol. On ajoute ensuite 10 ml de soude 1N. La réaction est complète en 1h30. On évapore les solvants sous vide et on acidifie la phase aqueuse résiduelle par addition d'acide chlorhydrique concentré jusqu'à obtention d'un pH de 1. On purifie le composé obtenu par solubilisation dans l'éthanol et précipitation dans l'eau sous agitation. La masse récupérée est de 0,05 g, ce qui correspond à un rendement de 25%.

Les analyses en RMN ¹H (200MHz ; DMSO) δ ppm ont donné les résultats suivants :
3.8 (3H ; s), 5.1 (2H ; s), 7 (3H ; t), 7.6 (2H ; d), 7.9 (2H ; d), 11.7 (1H ; s).
La structure du composé obtenu est conforme à la structure attendue.

### Exemple n°7 : synthèse de l'acide 3-H-benzo[E] indole-2-carboxylique :

### a) Synthèse de l'ester méthylique de l'acide 2-azido-3-naphtalène-1-yl-acrylique de formule :

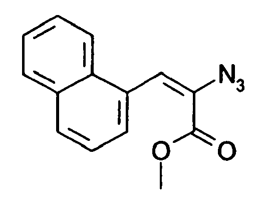

Dans un réacteur, on introduit 20 ml de méthylate de sodium en solution à 30%. On refroidit à O°C, puis on ajoute lentement 5 g de naphtaldéhyde sous argon. On ajoute ensuite goutte à goutte 8,3 g d'azido acétate d'éthyle, dilués dans 10 ml de méthanol. On laisse réagir 5 heures à O°C. On ajoute 50 ml d'eau à la réaction et on extrait par 2 x 30 ml de dichlorométhane. On sèche la phase organique sur sulfate de sodium et on évapore à sec sous vide et on purifie le produit obtenu sur colonne de silice en utilisant du dichlorométhane comme éluant. La masse récupérée est de 4 g ce qui correspond à un rendement de 70%.

### b) Synthèse de l'ester méthylique de l'acide 3-H-benzo [E] indole-2-carboxylique de formule :

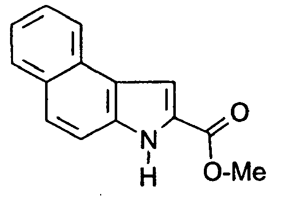

Dans un réacteur, on solubilise 3 g d'ester méthylique de l'acide 2-azido-3-naphtalène-1-yl-acrylique, dans 20 ml de toluène. On porte le mélange à reflux pendant 3 heures. On évapore le solvant sous vide puis on reprend le résidu dans 20 ml d'heptane chaud. On obtient un solide qui précipite. On filtre et on sèche le solide au dessiccateur sous vide. La masse récupérée est de 3,1 g ce qui correspond à un rendement de 89%.

### c) Synthèse de l'acide 3-H-benzo [E] indole-2-carboxylique de formule :

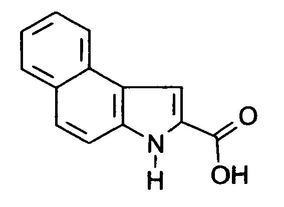

Dans un réacteur, on solubilise 1 g d'ester méthylique de l'acide 3-H-benzo [E] indole-2-carboxylique, dans 10 ml de méthanol et 20 ml d'acétone. On ajoute ensuite 50 ml de soude 1N. On laisse réagir 1h30 à température ambiante. On évapore les solvants sous vide et on acidifie la phase aqueuse résiduelle en ajoutant de l'acide chlorhydrique concentré jusqu'à obtention d'un pH de 1. On filtre le solide et on le recristallise dans un mélange eau/éthanol. La masse récupérée est de 0,5 g ce qui correspond à un rendement de 53%.

Les analyses en RMN ¹H (200MHz ; DMSO) δ ppm ont donné les résultats suivants :
7.75 (5H ; m), 8.1 (1H ; d), 8.5 (1H ; d), 12.3 (1H ; s), 13.1 (1H ; s)

| Analyse Elémentaire : | | | | |
|---|---|---|---|---|
| | C en % | H en % | N en % | O en % |
| théorique | 73.92 | 4.29 | 6,63 | 15.15 |
| trouvée | 73.58 | 4,23 | 6.52 | 15.15 |

### Exemple n°8 : Synthèse de l'acide 3-méthyl-3H-benzo [E] indole-2-carboxylique

### a) Synthèse de l'ester méthylique de l'acide 3-méthyl-3-H-benzo [E] indole-2-carboxylique de formule :

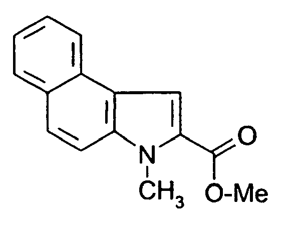

Dans un réacteur on introduit 1,1 équivalents d'hydrure de sodium (NaH) sous argon et 5 ml de diméthylformamide (DMF) anhydre. On ajoute 0,1 g d'ester méthylique de l'acide 3-H-benzo [E] indole-2-carboxylique et on laisse sous agitation jusqu'à obtention d'un milieu homogène. On ajoute ensuite 0,1 ml d'iodure de méthyle, et on laisse réagir à température ambiante. La réaction est complète en 30 minutes. On ajoute ensuite 30 ml d'une solution saturée en NaCl puis on extrait par deux fois 50 ml de dichlorométhane. On concentre la phase organique à sec sous vide. La masse récupérée est de 0,11 g ce qui correspond à un rendement de 100%.

### b) Synthèse de l'acide 3-méthyl-3-H-benzo [E] indole-2-carboxylique de formule:

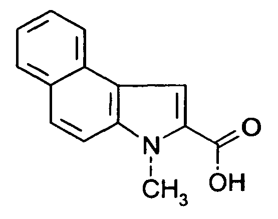

Dans un réacteur, on solubilise 0,1 g d'ester méthylique de l'acide 3-méthyl-3-H-benzo [E] indole-2-carboxylique dans 7 ml d'acétone. On ajoute ensuite 10 ml de soude 1N. On laisse réagir à température ambiante pendant 1 heure. On évapore le solvant sous vide et on neutralise la phase aqueuse résiduelle en portant le pH à 6 par addition d'acide chlorhydrique concentré. Le solide obtenu est lavé plusieurs fois par de l'eau.
La masse récupérée est de 0,057 g ce qui correspond à un rendement de 61%.

Les analyses en RMN ¹H (200MHz ; DMSO) δ ppm ont donné les résultats suivants :
7.35 (2H ; m), 7.7 (4H ; m), 8.2 (1H ; d), 12.7 (1H ; s)

### Exemple n°9 : synthèse de l'acide 1-(3,5-bis-trifluorométhyl-benzyl)-5-(3,5-bis-trifluorométhyl-benzyloxy)-6-méthoxy-1H-indole-2-carboxylique :

### a) Synthèse de l'ester éthylique de l'acide 1-(3,5-bis-trifluorométhyl-benzyl)-5-(3,5-bis-trifluorométhyl-benzyloxy)-6-méthoxy-1H-indole-2-carboxylique de formule :

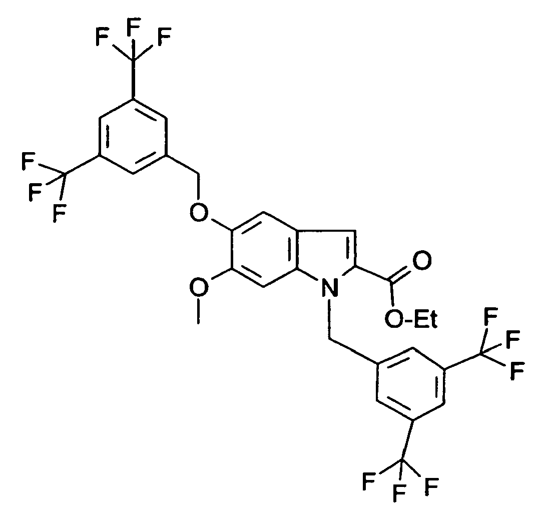

Dans un réacteur préalablement séché à l'étuve, on introduit 1,6 équivalents d'hydrure de sodium (NaH) sous argon et 5 ml de diméthylformamide (DMF) anhydre. On ajoute ensuite 0,3 g de l'ester éthylique de l'acide 5-hydroxy-6-méthoxy-1 H-indole-2-carboxylique à +3°C, et on laisse réagir 30 minutes à cette température afin de former l'alcoolate. On ajoute ensuite 1,4 équivalents de bromure de 3,5-bis-(trifluorométhyle)-benzyle et on laisse réagir à cette température pendant 1h30. On verse ensuite 50 ml d'une solution saturée en bicarbonate de sodium (NaHCO₃) et on extrait par deux fois 50 ml de dichlorométhane. On sèche la phase organique sur sulfate de sodium, on la concentre à sec sous vide et on purifie le résidu obtenu sur colonne de silice en utilisant le dichlorométhane comme éluant. La masse récupérée est de 200 mg ce qui correspond à un rendement de 40%.

### b) Synthèse de l'acide 1-(3,5-bis-trifluorométhyl-benzyl)-5-(3,5-bis-trifluorométhyl-benzyloxy)-6-méthoxy-1 H-indole-2-carboxylique de formule :

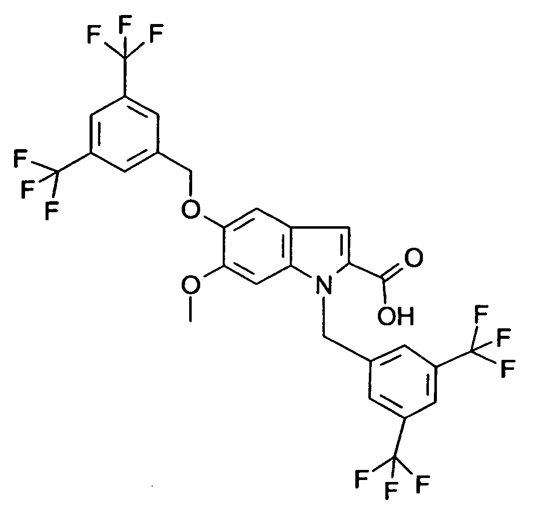

Dans un réacteur, on solubilise 0,2 g d'ester éthylique de l'acide 1-(3,5-bis-trifluorométhyl-benzyl)-5-(3,5-bis-trifluorométhyl-benzyloxy)-6-méthoxy-1 H-indole-2-carboxylique dans 10 ml d'acétone et 5 ml de méthanol. On ajoute ensuite 10 ml de soude 1N. On laisse réagir 2 heures à température ambiante. On évapore sous vide les solvants et on acidifie la phase aqueuse résiduelle par addition d'acide chlorhydrique concentré jusqu'à obtention d'un pH de 3. On purifie par reprise dans l'éther isopropylique. La masse récupérée est de 0,08 g ce qui correspond à un rendement de 45%.

Les analyses en RMN ¹H (200MHz ; DMSO) δ ppm ont donné les résultats suivants :
3.8 (3H ; s), 5.3 (2H ; s), 6.2 (2H ; s), 7.2 (3H ; t), 8.0 (6H ; m)
La structure du composé obtenu est conforme à la structure attendue.

### Exemple n°10 : évaluation de l'effet inhibiteur de composés de l'invention sur les 5α-réductases de types 1 et 2 :

Les séquences codantes (Acides désoxyribonlucléiques complémentaires : cDNA) de la 5α-réductase 1 et de la 5α-réductase 2 ont été clonées dans le vecteur d'expression eucaryote pSG5 (Stratagene). Les enzymes ont été surexprimées après transfection transitoire de cellules COS7 (ATCC, CRL1651).

Les cDNAs de la 5α-réductase 1 et de la 5α-réductase 2 ont été obtenus par transcription inverse et réaction de polymérisation en chaîne (Polymerase chain reaction : PCR) à l'aide d'amorces spécifiques à partir d'ARN total de testicules humains (vendu par la société Clontech).
Les amorces utilisées pour obtenir le cDNA de la 5α-réductase 1 sont :
Brin + : 5' CCCAGCCCTGGCGATGGCAAC 3',
Brin - : 5' GGATATTCAACCTCCATTTCAG 3'.
Les amorces utilisées pour obtenir le cDNA de la 5α-réductase 2 sont :
Brin + : 5' GCGATGCAGGTTCAGTG 3',
Brin - : 5' ATTGTGGGAGCTCTGCT 3'.

Par des techniques classiques de génie génétique, le cDNA de la 5α-réductase 1 obtenu a été inséré au site BamHI de pSG5 et celui de la 5α-réductase 2 au site EcoRI (voir Magnatis et col., Molecular Cloning, Cold Spring Harber, 1989)

Les clones positifs (recombinants) ont été identifiés par la technique d'hybridation avec une sonde froide (Plex luminescent kits, Millipore) et cartographiés par digestions enzymatiques et séquençage partiel.
Après transfection transitoire les cellules COS7 sont lysées dans un tampon Tris-HCI 10 mM, pH=7 / 150 mM KCI / 1 mM EDTA par 3 cycles de congélation/décongélation. L'homogénat est centrifugé à 100 000 g pendant 1 heure. Les culots contenant la 5α-réductase 1 ou la 5α-réductase 2 sont repris dans un tampon phosphate 40 mM, pH 6.5 ou citrate 40 mM, pH 5.5 respectivement pour la 5α-réductase 1 ou 5α-réductase 2.
5 µg de protéines ainsi obtenues sont incubés dans un puits d'une plaque 96 puits (NUNC) en présence de 1 nM de ¹⁴C-Testostérone (Amersham) et de 5 mM de Nicotinamide Adenosine Dinucleotide Phosphate, forme réduite (NADPH) (Sigma) dans le tampon correspondant (tampon phosphate 40 mM, pH 6.5 ou citrate 40 mM, pH 5.5 respectivement pour la 5α-réductase 1 ou 5α-réductase 2) pendant 50 minutes à 37°C après addition des produits à tester.
Les produits à tester sont ajoutés aux concentrations de 10⁻⁴ M à 10⁻¹⁰M, dilués dans du tampon phosphate 40 mM, pH 6.5 ou citrate 40 mM, pH 5.5 respectivement pour la 5α-réductase 1 ou 5α-réductase 2.
Les mélanges réactionnels sont ensuite déposés directement sur un plaque de silice (HPTLC, 60F 254, Merck) et soumis à une chromatographie (solvant = 10% diéthyléther, 90% dichlorométhane). Ils sont ensuite analysés par autoradiographie digitale (Digital Autoradiography, Berthold).
On mesure l'inhibition de l'activité des isoenzymes en calculant le pourcentage de dihydrotestostérone formée à partir de ¹⁴C-testostérone par rapport à un témoin non traité.
Les résultats sont présentés dans le tableau suivant :

| | | | | | | 5α-réductases : IC50 | |
|---|---|---|---|---|---|---|---|
| ex | X | Y | Z | R₁ | R₂ | Type I (µm) | Type II (nM) |
| 1 | OCH₂Ph | H | - OMe | H | H | 1-4 | >100 000 |
| 2 | OCH₂Ph(CF₃)₂ | H | -OMe | H | H | 10 | >100 000 |
| 3 | H | OCH₂Ph(CF₃)₂ | -OMe | H | H | 0.5 | 1-5 |
| 4 | H | OCH₂Ph(OMe)₃ | -OMe | H | H | >100 | 10 |
| 5 | H | OCH₂Ph (OMe) | -OMe | H | H | 1-5 | 10 |
| 6 | H | OCH₂Ph(CN) | -OMe | H | H | 5 | 40 |
| 7 | Ph | | H | H | H | 3 | 6000 |
| 8 | Ph | | H | H | Me | 0,5 | 10000 |
| a | H | OCH₂Ph | -OMe | H | H | 1 | 1 |
| b | H | OCH₂Ph | -OCH₂Ph | Me | H | >100 | 1000 |
| c | H | OCH₂Ph | -OCH₂Ph | H | H | >100 | 5 |

| | | | | | | | |
|---|---|---|---|---|---|---|---|
| Ph = -C₆H₅ ; Me = -CH₃ | | | | | | | |
| a, b, c : composés dont la synthèse n'est pas exemplifiée. | | | | | | | |

Les composés des exemples 1 et 2 présentent une spécificité très marquée vis-à-vis de la 5α-réductase de type 1.
Les composés des exemples 7 et 8 présentent une spécificité marquée vis-à-vis de la 5α-réductase de type 1.
Les composés des exemples 3, 5, 6 et le composé a sont de très bons inhibiteurs des deux types de 5α-réductase.

Le composé de l'exemple 4 et le composé c présentent une spécificité plus marquée pour la 5α-réductase de type 2.

Le composé b est un inhibiteur moyen avec une spécificité plus marquée pour le type I.

### Exemple n°10 : Exemples de compositions contenant un dérivé indole-carboxylique.

Ces compositions sont obtenues par les techniques habituelles couramment utilisées en cosmétique ou en pharmacie.

| Lotion antichute : | | |
|---|---|---|
| Composé de l'exemple 3 | | 2,000 g |
| Propylène glycol | | 30,000 g |
| Alcool éthylique | | 40,500 g |
| Eau | qsp | 100,000 g |

On applique cette lotion sur le cuir chevelu, une à deux fois par jour, à raison d'1 ml par application.

| lotion antichute : | | |
|---|---|---|
| Composé de l'exemple 3 | | 2,500 g |
| Monométhyléther de propylèneglycol (Dowanol PM de Dow Chemical) | | 20,000 g Hydrox |
| Alcool éthylique | | 40,000 g |
| Minoxidil | | 2,000 g |
| Eau | qsp | 100,000 g |

On applique cette lotion épaissie sur le cuir chevelu, une à deux fois par jour, à raison d'1 ml par application.

Avec chacune des compositions décrites dans les exemples ci-dessus, on a constaté, après plusieurs mois de traitement et selon les sujets traités, un ralentissement de la chute des cheveux et/ou un effet repousse.

## Revendications

1. Utilisation d'une quantité efficace d'au moins un composé répondant à la formule générale (I) 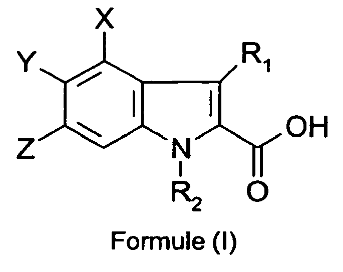 dans laquelle :
X représente un atome d'hydrogène ;
Y représente un radical -O-CHR₃R₄; dans lequel R₃ est un atome d'hydrogène ou un radical phényle éventuellement substitué ou encore un hétérocycle ayant 5 ou 6 chaînons et R₄ est un radical phényle éventuellement substitué ou encore un hétérocycle ayant 5 ou 6 chaînons, ou R₃ et R₄ pris ensemble forment avec l'atome de carbone un cycle ou un hétérocycle ayant 5 ou 6 chaînons ;
Z représente un radical -O-R₅ dans lequel R₅ est un radical alkyle en C₁-C₄ choisi parmi les radicaux méthyle, éthyle, propyle, isopropyle, butyle, isobutyle ou tertiobutyle;
R₁ représente un atome d'hydrogène;
R₂ représente un atome d'hydrogène;
étant entendu que lorsque R₃ ou R₄ indépendamment l'un de l'autre sont un radical phényle substitué, celui-ci est substitué par un groupement cyano (-CN), un groupement trifluorométhyle (-CF₃), un radical méthoxy (-O-CH₃) ou un atome d'halogène,
pour la fabrication d'une composition destinée au traitement des désordres liés à une suractivité de la 5α-réductase de type 2, lesdits désordres étant choisis parmi l'acné, l'hirsutisme, la séborrhée, l'alopécie androgénique, les carcinomes prostatiques et l'hyperplasie bénigne de la prostate.

2. Utilisation d'une quantité efficace d'au moins un composé choisi parmi :
l'acide 5-(3,4,5-triméthoxy-benzyloxy)-6-méthoxy -1H-indole-2-carboxylique ;
l'acide 5-(4-méthoxy-benzyloxy)-6-méthoxy-1 H-indole-2-carboxylique ;
l'acide 5-(4-cyano-benzyloxy)-6-méthoxy-1H-indole-2-carboxylique ;
l'acide 1-(3,5-bis-trifluorométhyl-benzyl)-5-(3,5-trifluorométhyl-benzyloxy)-6-méthoxy-1H-indole-2-carboxylique ;
l'acide 5-[3,5-bis(trifluoromethyl)benzyloxy]-6-méthoxy-1H-indole-2-carboxylique; un dérive selon la formule (I) de la revendication 1 pour lequel Y représente un radical -OCH₂Ph, Z représente un radical -OMe, R₁ et R₂ représentent un atome d'hydrogène;
un dérivé pour lequel Y et Z représentent un radical -OCH₂Ph, R₁ et R₂ représente un hydrogène,
pour la fabrication d'une composition destinée au traitement des désordres liés à une suractivité de la 5α-réductase de type 2, lesdits désordres étant choisis parmi l'acné, l'hirsutisme, la séborrhée, l'alopécie androgénique, les carcinomes prostatiques et l'hyperplasie bénigne de la prostate.

3. Utilisation selon l'une quelconque des revendications 1 ou 2, caràctéilsée par **le fait que** le composé ou la composition sont destinés à induire et/ou stimuler la croissance des cheveux et/ou freiner leur chute et/ou à traiter l'hyperséborrhée et/ou l'acné.

4. Utilisation selon l'une quelconque des revendications 1 à 3, **caractérisée par le fait que** le composé est utilisé en une quantité représentant de 0,001% à 10% du poids total de la composition.

5. Utilisation selon la revendication 4, **caractérisée par le fait que** le composé est utilisé en une quantité représentant de 0,01% à 5% du poids total de la composition.

6. Procédé de traitement cosmétique de la peau, des cheveux et/ou du cuir chevelu, **caractérisé par le fait qu'**il consiste à appliquer sur les cheveux et/ou le cuir chevelu et/ou sur la peau, une composition cosmétique comprenant un composé de formule (I') telle que définie ci-dessous dans l'une quelconque des revendications 10 à 12, à laisser celle-ci au contact de la peau, des cheveux et/ou du cuir chevelu, et éventuellement à rincer.

7. Dérivé indole-carboxylique répondant à la formule générale (I') : 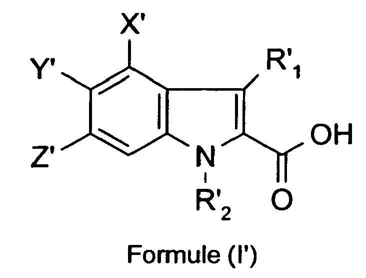 dans laquelle :
X' représente un atome d'hydrogène
Y' représente un radical -O-CHR'₃R'₄, dans lequel R'₃ est un atome d'hydrogène ou un radical phényle éventuellement substitué ou encore un hétérocycle ayant 5 ou 6 chaînons et R'₄ est un radical phényle éventuellement substitué ou encore un hétérocycle ayant 5 ou 6 chaînons, ou R'₃ et R'₄ pris ensemble forment avec l'atome de carbone un cycle ou un hétérocycle ayant 5 ou 6 chaînons ;
Z' représente un radical -O-R'₅ dans lequel R'₅ est un radical alkyle en C₁-C₄ choisi parmi les radicaux méthyle, éthyle, propyle, isopropyle, butyle, isobutyle ou tertiobutyle;
R'₁ représente un atome d'hydrogène;
R'₂ représente un atome d'hydrogène;
ou ses esters ou ses isomères optiques, seuls ou en mélange en toutes proportions encore ses sels pharmaceutiquement acceptables.
étant entendu :
que Y' ne peut être un radical -OCHR'₃R'₄ avec -CHR'₃R'₄ représentant un radical aralkyle, et que lorsque R'₃ ou R'₄ indépendamment l'un de l'autre sont un radical phényle substitué, celui-ci est substitué par un groupement cyano (-CN), un groupement trifluorométhyle (-CF₃), un radical méthoxy (-O-CH₃) ou un atome d'halogène.

8. Dérivé selon la revendication 7, **caractérisé par le fait que** quand R'₃ ou R'₄ indépendamment l'un de l'autre sont un hétérocycle celui-ci est choisi parmi la pyridine, la quinoline, l'imidazole, le benzimidazole, le tétrahydrofuranne ou le furanne.

9. Dérivé choisi parmi :
l'acide 5-(3,4,5-triméthoxy-benzyloxy)-6-méthoxy -1 H-indole-2-carboxylique,
l'acide 5-(4-méthoxy-benzyloxy)-6-méthoxy-1 H-indole-2-carboxylique,
l'acide 5-(4-cyano-benzyloxy)-6-méthoxy-1H-indole-2-carboxylique,
l'acide 1-(3,5-bis-trifluorométhyl-benzyl)-5-(3,5-trifluorométhyl-benzyloxy)-6-méthoxy-1 H-indole-2-carboxylique,
l'acide 5-[3,5-bis(trifluoromethyl) benzyloxy]-6-méthoxy-1H-indole-2-carboxylique, un dérivé selon la formule (1') de la revendication 7 pour lequel Y' représente un radical -OCH₂Ph, Z' représente un radical -OMe, R'₁ et R'₂ représentent un atome d'hydrogène;
un dérivé pour lequel Y' et Z' représentent un radical -OCH₂Ph, R'₁ et R'₂ représente un hydrogène.

10. Composition comprenant au moins un composé tel que défini selon l'une quelconque des revendications 7 à 9.

11. Composition selon ia revendication 10, **caractérisée par le fait qu'**elle est destinée à un usage cosmétique ou pharmaceutique.

12. Composition selon l'une quelconque des revendications 10 ou 11, **caractérisée par le fait qu'**elle comprend au moins un composé tel que défini dans l'une des revendications 7 à 9 en une quantité représentant de 0,001% à 10% du poids total de la composition et préférentiellement en une quantité représentant de 0,01% à 2% du poids total de la composition.

13. Composition selon l'une quelconque des revendications 10 à 12, **caractérisée par le fait qu'**elle comprend également au moins un agent choisi parmi les agents antibactériens, les antiparasitaires, les antifongiques, les antiviraux, les anti-inflammatoires, les antiprurigineux, les anesthésiques, les kératolytiques, les anti-radicaux libres, les anti-séborrhéiques, les antipelliculaires, les antiacnéiques et/ou les agents diminuant la différenciation et/ou la prolifération et/ou la pigmentation cutanée, les extraits d'origine végétale ou bactérienne.

14. Composition selon l'une quelconque des revendications 10 à 13,
**caractérisée par le fait qu'**elle est destinée à traiter les désordres liés à une suractivité de la 5α-réductase de type 2.

15. Composition selon l'une quelconque des revendications 10 à 14, **caractérisée par le fait qu'**elle est destinée à traiter les désordres androgéno-dépendants.

16. Composition selon l'une quelconque des revendications 10 à 15, **caractérisée par le fait qu'**elle est destinée à traiter la séborrhée et/ou l'acné et/ou l'hirsutisme et/ou l'alopécie androgénétique.

17. A titre de médicaments les composés tels que définis dans les revendications 7 à 9.

## Claims

1. Use of an effective amount of at least one compound corresponding to the general formula (I) 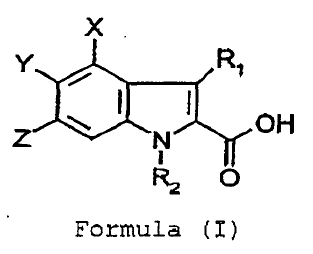 in which:
X represents a hydrogen atom;
Y represents a radical -O-CHR₃R₄, in which R₃ is a hydrogen atom or an optionally substituted phenyl radical or alternatively a 5- or 6-membered heterocycle and R₄ is an optionally substituted phenyl radical or alternatively a 5- or 6-membered heterocycle, or R₃ and
R₄ taken together form, with the carbon atom, a 5- or 6-membered ring or heterocycle;
Z represents a radical -O-R₅ in which R₅ is a C₁-C₄ alkyl radical chosen from the methyl, ethyl, propyl, isopropyl, butyl, isobutyl or tert-butyl radicals;
R₁ represents a hydrogen atom;
R₂ represents a hydrogen atom;
it being understood that when R₃ or R₄, independently of each other, is a substituted phenyl radical, this radical is substituted with a cyano group (-CN), a trifluoromethyl group (-CF₃), a methoxy radical (-O-CH₃) or a halogen atom,
for the manufacture of a composition for treating disorders associated with overactivity of type-2 5α-reductase, the said disorders being chosen from acne, hirsutism, seborrhoea, androgenic alopecia, prostate carcinomas and benign hyperplasia of the prostate.

2. Use of an effective amount of at least one compound chosen from:
5-(3,4,5-trimethoxybenzyloxy)-6-methoxy-1H-indole-2-carboxylic acid;
5-(4-methoxybenzyloxy)-6-methoxy-1H-indole-2-carboxylic acid;
5-(4-cyanobenzyloxy)-6-methoxy-1H-indole-2-carboxylic acid;
1-[3, 5-bis (trifluoromethyl)benzyl]-5-[3,5-bis (trifluoromethyl)benzyloxy]-6-methoxy-1H-indole-2-carboxylic acid;
5-[3,5-bis(trifluoromethyl)benzyloxy]-6-methoxy-1H-indole-2-carboxylic acid;
a derivative according to formula (I) of Claim 1 for which Y represents an -OCH₂Ph radical, Z represents an -OMe radical and R₁ and R₂ represent a hydrogen atom;
a derivative for which Y and Z represent an -OCH₂Ph radical and R₁ and R₂ represent a hydrogen atom; for the manufacture of a composition for treating disorders associated with overactivity of type-2 5α-reductase, the said disorders being chosen from acne, hirsutism, seborrhoea, androgenic alopecia, prostate carcinomas and benign hyperplasia of the prostate.

3. Use according to either of Claims 1 and 2, **characterized in that** the compound or the composition is intended to induce and/or stimulate hair growth and/or to slow down hair loss and/or to treat hyper-seborrhoea and/or acne.

4. Use according to any one of Claims 1 to 3, **characterized in that** the compound is used in an amount representing from 0.001% to 10% of the total weight of the composition.

5. Use according to Claim 4, **characterized in that** the compound is used in an amount representing from 0.01% to 5% of the total weight of the composition.

6. Cosmetic treatment process for the skin and/or the hair and/or the scalp, **characterized in that** it consists in applying to the hair and/or the scalp and/or the skin a cosmetic composition comprising a compound of formula (I') as defined below in any one of Claims 10 to 12, in leaving this composition in contact with the skin and/or the hair and/or the scalp, and optionally in rinsing it out.

7. Indolecarboxylic derivative corresponding to the general formula (I'): 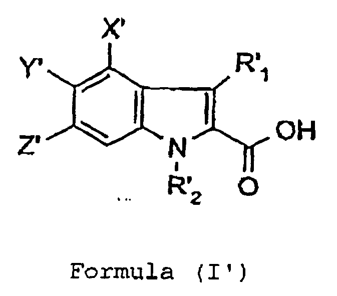 in which:
X' represents a hydrogen atom
Y' represents a radical -O-CHR'₃R'₄, in which R'₃ is a hydrogen atom or an optionally substituted phenyl radical or alternatively a 5- or 6-membered heterocycle and R'₄ is an optionally substituted phenyl radical or alternatively a 5- or 6-membered heterocycle, or R'₃ and
R'₄ taken together form, with the carbon atom, a 5- or 6-membered ring or heterocycle;
Z represents a radical -O-R'₅ in which R'₅ is a C₁-C₄ alkyl radical chosen from the methyl, ethyl, propyl, isopropyl, butyl, isobutyl or tert-butyl radicals;
R'₁ represents a hydrogen atom;
R'₂ represents a hydrogen atom;
or the esters thereof or the optical isomers thereof, alone or as a mixture in all proportions, or alternatively the pharmaceutically acceptable salts thereof,
it being understood:
that Y' cannot be a radical -OCHR'₃R'₄ with -CHR'₃R'₄ representing an aralkyl radical, and that, when R'₃ or R'₄, independently of each other, is an optionally substituted phenyl radical, this radical is substituted with a cyano (-CN) group, a trifluoromethyl (-CF₃) group, a methoxy (-O-CH₃) radical or a halogen atom.

8. Derivative according to Claim 7, **characterized in that**, when R'₃ or R'₄, independently of each other, is a heterocycle, this heterocycle is chosen from pyridine, quinoline, imidazole, benzimidazole, tetrahydrofuran and furan.

9. Derivative chosen from:
5-(3,4,5-trimethoxybenzyloxy)-6-methoxy-1H-indole-2-carboxylic acid,
5-(4-methoxybenzyloxy)-6-methoxy-1H-indole-2-carboxylic acid,
5-(4-cyanobenzyloxy)-6-methoxy-1H-indole-2-carboxylic acid,
1-[3,5-bis(trifluoromethyl)benzyl]-5-[3,5-bis-(trifluoromethyl)benzyloxy]-6-methoxy-1H-indole-2-carboxylic acid,
5-[3,5-bis(trifluoromethyl)benzyloxy]-6-methoxy-1H-indole-2-carboxylic acid;
a derivative according to formula (I') of Claim 7 for which Y' represents an -OCH₂Ph radical, Z' represents an -OMe radical and R'₁ and R'₂ represent a hydrogen atom; a derivative for which Y' and Z' represent an -OCH₂Ph radical and R'₁ and R'₂ represent a hydrogen atom.

10. Composition comprising at least one compound as defined according to any one of Claims 7 to 9.

11. Composition according to Claim 10, **characterized in that** it is intended for cosmetic or pharmaceutical use.

12. Composition according to either of Claims 10 and 11, **characterized in that** it comprises at least one compound as defined in one of Claims 7 to 9 in an amount representing from 0.001% to 10% of the total weight of the composition, and preferably in an amount representing from 0.01% to 2% of the total weight of the composition.

13. Composition according to any one of Claims 10 to 12, **characterized in that** it also comprises at least one agent chosen from antibacterial agents, antiparasitic agents, antifungal agents, antiviral agents, anti-inflammatory agents, anti-pruriginous agents, anaesthetics, keratolytic agents, anti-free-radical agents, anti-seborrhoeic agents, antidandruff agents, anti-acne agents and/or agents for reducing skin differentiation and/or proliferation and/or pigmentation, and extracts of plant or bacterial origin.

14. Composition according to any one of Claims 10 to 13, **characterized in that** it is intended to treat disorders associated with overactivity of type-2 5α-reductase.

15. Composition according to any one of Claims 10 to 14, **characterized in that** it is intended to treat androgen-dependent disorders.

16. Composition according to any one of Claims 10 to 15, **characterized in that** it is intended to treat seborrhoea and/or acne and/or hirsutism and/or androgenic alopecia.

17. As medicinal compounds, the compounds as defined in Claims 7 to 9.

## Patentansprüche

1. Verwendung einer wirksamen Menge mindestens einer Verbindung, die der allgemeinen Formel (I) entspricht 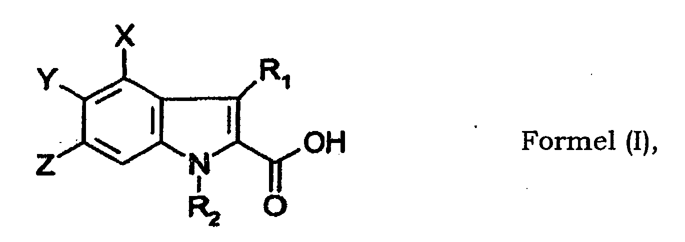 worin bedeuten:
X ein Wasserstoffatom;
Y eine Gruppe -O-CHR₃R₄, wobei R₃ ein Wasserstoffatom oder eine gegebenenfalls substituierte Phenylgruppe oder einen 5-oder 6-gliedrigen Heterocyclus bedeutet und R₄ eine gegebenenfalls substituierte Phenylgruppe oder ein 5- oder 6-gliedriger Heterocyclus ist, wobei R₃ und R₄ gemeinsam mit dem Kohlenstoff atom einen Ring oder Heterocyclus mit 5 oder 6 Ringbestandteilen bilden können;
Z eine Gruppe -O-Rs, wobei R₅ eine C₁₋₄-Alkylgruppe ist, die unter Methyl, Ethyl, Propyl, Isopropyl, Butyl, Isobutyl oder *t*-Butyl ausgewählt ist;
R₁ ein Wasserstoffatom;
R₂ ein Wasserstoffatom;
mit der Maßgabe, dass, wenn die Gruppen R₃ oder R₄ unabhängig voneinander eine substituierte Phenylgruppe bedeuten, diese mit einer Cyanogruppe (-CN), einer Trifluormethylgruppe (-CF₃), einer Methoxygruppe (-O-CH₃) oder einem Halogenatom substituiert ist,
für die Herstellung einer Zusammensetzung, die für die Behandlung von Störungen vorgesehen ist, die mit einer übermäßigen Aktivität der 5α-Reduktase vom Typ 2 zusammenhängen, wobei diese Störungen unter Akne, Hirsutismus, Seborrhoe, androgener Alopezie, Prostatakarzinomen und benigner Hyperplasie der Prostata ausgewählt sind.

2. Verwendung einer wirksamen Menge mindestens einer Verbindung, die unter den folgenden Verbindungen ausgewählt ist:
5-(3,4,5-Trimethoxy-benzyloxy)-6-methoxy-1H-indol-2-carbonsäure;
5-(4-Methoxy-benzyloxy)-6-methoxy-1 H-indol-2-carbonsäure;
5-(4-Cyano-benzyloxy)-6-methoxy-1H-indol-2-carbonsäure;
1-(3,5-Bis-triflurmethyl-benzyl)-5-(3,5-trifluormethyl-benzyloxy)-6-methoxy-1 H-indol-2-carbonsäure;
5- [3, 5-Bis(trifluormethyl)benzyloxy]-6-methoxy-1 H-indol-2-carbonsäure;
ein Derivat gemäß Formel (I) nach Anspruch 1, worin Y die Gruppe -OCH₂Ph, Z die Gruppe -OMe und R₁ und R₂ ein Wasserstoffatom bedeuten;
ein Derivat, worin Y und Z die Gruppe -OCH₂Ph bedeuten und R₁ und R₂ Wasserstoff bedeutet,
für die Herstellung einer Zusammensetzung, die für die Behandlung von Störungen vorgesehen ist, die mit einer übermäßigen Aktivität der 5α-Reduktase vom Typ 2 zusammenhängen, wobei diese Störungen unter Akne, Hirsutismus, Seborrhoe, androgener Alopezie, Prostatakarzinomen und benigner Hyperplasie der Prostata ausgewählt sind.

3. Verwendung nach einem der Ansprüche 1 oder 2, **dadurch gekennzeichnet, dass** die Verbindung oder Zusammensetzung dazu vorgesehen sind, das Wachstum der Haare zu induzieren und/oder zu stimulieren und/oder den Haarausfall zu verlangsamen und/oder Hyperseborrhoe und/oder Akne zu behandeln.

4. Verwendung nach einem der Ansprüche 1 bis 3, **dadurch gekennzeichnet, dass** die Verbindung in einer Menge von 0,001 bis 10 % des Gesamtgewichts der Zusammensetzung verwendet wird.

5. Verwendung nach Anspruch 4, **dadurch gekennzeichnet, dass** die Verbindung in einer Menge von 0,01 bis 5 % des Gesamtgewichts der Zusammensetzung verwendet wird.

6. Verfahren zur kosmetischen Behandlung der Haut, der Haare und/oder der Kopfhaut, **dadurch gekennzeichnet, dass** es darin besteht, auf die Haare und/oder die Kopfhaut und/oder die Haut eine kosmetische Zusammensetzung aufzutragen, die eine Verbindung der Formel (I'), wie sie nachstehend in einem der Ansprüche 10 bis 12 definiert ist, enthält, diese in Kontakt mit der Haut, den Haaren und/oder der Kopfhaut zu belassen und gegebenenfalls zu spülen.

7. Indolcarbonsäurederivate der allgemeinen Formel (I'): 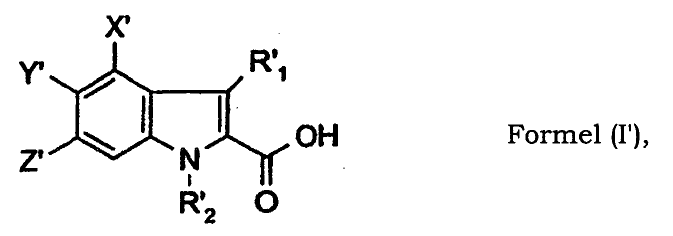 worin bedeuten:
X' ein Wasserstoffatom;
Y' eine Gruppe -O-CHR'₃R'₄, wobei R'₃ ein Wasserstoffatom oder eine gegebenenfalls substituierte Phenylgruppe oder einen 5-oder 6-gliedrigen Heterocyclus bedeutet und R'₄ eine gegebenenfalls substituierte Phenylgruppe oder ein 5- oder 6-gliedriger Heterocyclus ist, wobei R'₃ und R'₄ gemeinsam mit dem Kohlenstoffatom einen Ring oder Heterocyclus mit 5 oder 6 Ringbestandteilen bilden können;
Z eine Gruppe -O-R'₅, wobei R'₅ eine C₁₋₄-Alkylgruppe ist, die unter Methyl, Ethyl, Propyl, Isopropyl, Butyl, Isobutyl oder *t*-Butyl ausgewählt ist;
R'₁ ein Wasserstoffatom;
R'₂ ein Wasserstoffatom;
oder ihre Ester oder optischen Isomere, einzeln oder im Gemisch in beliebigen Mengenanteilen, oder ihre pharmazeutisch akzeptablen Salze,
mit der Maßgabe: dass Y' keine Gruppe -OCHR'₃R'₄ bedeuten kann, worin -CHR'₃R'₄ eine Aralkylgruppe bedeutet, und, wenn
die Gruppen R'₃ oder R'₄ unabhängig voneinander eine substituierte Phenylgruppe bedeuten, diese mit einer Cyanogruppe (-CN), einer Trifluormethylgruppe (-CF₃), einer Methoxygruppe (-O-CH₃) oder einem Halogenatom substituiert ist.

8. Derivat nach Anspruch 7, **dadurch gekennzeichnet, dass**, wenn die Gruppen R'₃ oder R'₄ unabhängig voneinander einen Heterocyclus bedeuten, dieser unter Pyridin, Chinolin, Imidazol, Benzimidazol, Tetrahydrofuran oder Furan ausgewählt ist.

9. Derivat, das ausgewählt ist unter:
- 5-(3,4,5-Trimethoxy-benzyloxy)-6-methoxy-1H-indol-2-carbonsäure;
- 5-(4-Methoxy-benzyloxy)-6-methoxy-1H-indol-2-carbonsäure;
- 5-(4-Cyano-benzyloxy)-6-methoxy-1H-indol-2-carbonsäure;
- 1-(3,5-Bis-triflurmethyl-benzyl)-5-(3,5-trifluormethyl-benzyloxy)-6-methoxy-1 H-indol-2-carbonsäure;
- 5-[3,5-Bis(trifluormethyl)benzyloxy]-6-methoxy-1H-indol-2-carbonsäure;
- ein Derivat gemäß Formel (I') nach Anspruch 7, worin Y' die Gruppe -OCH₂Ph, Z' die Gruppe -OMe und R'₁ und R'₂ ein Wasserstoffatom bedeuten;
ein Derivat, worin Y' und Z' die Gruppe -OCH₂Ph bedeuten und R'₁ und R'₂ Wasserstoff bedeuten.

10. Zusammensetzung, die mindestens eine Verbindung nach einem der Ansprüche 7 bis 9 enthält.

11. Zusammensetzung nach Anspruch 10, **dadurch gekennzeichnet, dass** sie für eine kosmetische oder pharmazeutische Verwendung vorgesehen ist.

12. Zusammensetzung nach einem der Ansprüche 10 oder 11, **dadurch gekennzeichnet, dass** sie mindestens eine Verbindung nach einem der Ansprüche 7 bis 9 in einer Menge von 0,001 bis 10 % des Gesamtgewichts der Zusammensetzung und vorzugsweise in einer Menge von 0,01 bis 2 % des Gesamtgewichts der Zusammensetzung enthält.

13. Zusammensetzung nach einem der Ansprüche 10 bis 12, **dadurch gekennzeichnet, dass** sie ferner mindestens einen Wirkstoff enthält, der unter den antibakteriellen Wirkstoffen, antiparasitären Wirkstoffen, antimykotischen Wirkstoffen, antiviralen Wirkstoffen, entzündungshemmenden Wirkstoffen, juckreizstillenden Wirkstoffen, Anaesthetika, Keratolytika, Radikalfängern für freie Radikale, Wirkstoffen gegen Seborrhoe, Antischuppenmitteln, gegen Akne wirkenden Wirkstoffen und/oder Wirkstoffen, die die Differenzierung und/oder Prolieferation und/oder Pigmentierung der Haut vermindern, und Extrakten pflanzlicher oder bakterieller Herkunft ausgewählt ist.

14. Zusammensetzung nach einem der Ansprüche 10 bis 13, **dadurch gekennzeichnet, dass** sie für die Behandlung von Störungen vorgesehen ist, die mit einer übermäßigen Aktivität der 5α-Reduktase vom Typ 2 zusammenhängen.

15. Zusammensetzung nach einem der Ansprüche 10 bis 14, **dadurch gekennzeichnet, dass** sie für die Behandlung von androgenabhängigen Störungen vorgesehen ist.

16. Zusammensetzung nach einem der Ansprüche 10 bis 15, **dadurch gekennzeichnet, dass** sie für die Behandlung von Seborrhoe und/oder Akne und/oder Hirsutismus und/oder androgenetischer Alopezie vorgesehen ist.

17. Verbindungen nach einem der Ansprüche 7 bis 9 als Arzneimittel.
